# EUROPEAN PATENT APPLICATION

(11) **EP 4 691 358 A1**
(43) Date of publication of application: **11.02.2026**
(21) Application number: 24887616.1
(22) Date of filing: 06.09.2024
(51) Int. Cl.: A61B 5/0245

(54) **DETECTION DEVICE AND ELECTRONIC EQUIPMENT**

(30) Priority: 07.11.2023 CN 202311479145
(71) Applicant: Huawei Technologies Co., Ltd., Shenzhen, Guangdong 518129 (CN)
(72) Inventor: CHEN, Minli, Shenzhen, Guangdong 518129 (CN); DONG, Yongjiang, Shenzhen, Guangdong 518129 (CN); LOU, Xinxin, Shenzhen, Guangdong 518129 (CN); ZHANG, Chaolong, Shenzhen, Guangdong 518129 (CN); WEI, Xiaosong, Shenzhen, Guangdong 518129 (CN)
(74) Representative: Epping - Hermann - Fischer
(86) International application number: PCT/CN2024/117552
(87) International publication number: WO 2025/097997

(57) **Abstract**

Embodiments of this application relate to the field of health data detection technologies, and provide a detection apparatus and an electronic device, to resolve a problem that accuracy of health detection data is relatively low due to a complex tissue structure of a to-be-monitored part of a user. In the detection apparatus, a first photodetector is disposed between two adjacent first light sources, and second light sources are located in a detection region enclosed by a plurality of first light sources and a plurality of first photodetectors. The first light source has a first distance H1 and a second distance H2 from at least two of all first photodetectors, and the second light source has a third distance H3 and a fourth distance H4 from at least two of all first photodetectors. The first light sources, the second light sources, and the first photodetectors are distributed in distribution regions of the detection apparatus. Each light source and one photodetector may form one PPG module, and an effective detection depth in skin may vary with a distance between the light source and the photodetector.

## Description

This application claims priority to Chinese Patent Application No. 202311479145.X, filed with the China National Intellectual Property Administration on November 7, 2023 and entitled "DETECTION APPARATUS AND ELECTRONIC DEVICE", which is incorporated herein by reference in its entirety.

### TECHNICAL FIELD

This application relates to the field of health data detection technologies, and in particular, to a detection apparatus and an electronic device.

### BACKGROUND

As people pay more attention to health, users have increasingly high requirements for functions of electronic devices. A device with a human health sign detection function may detect health sign data of a user, such as a heart rate and blood oxygen, so that the user can learn of a physical condition of the user at any time, thereby preventing a disease. Currently, a finger of the user may be clamped by a finger-clip oximeter, to perform blood oxygen detection on a finger part. However, in the foregoing detection manner, a fingertip of the user cannot normally move and has a squeezing sensation, and it is difficult to implement real-time detection. To resolve the foregoing problem, a wearable device may be used to perform blood oxygen detection on another part of the user, for example, a wrist part. However, because a tissue structure of the wrist part is more complex than a tissue structure of the fingertip, accuracy of blood oxygen detection is reduced.

### SUMMARY

This application provides a detection apparatus and an electronic device, to resolve a problem that accuracy of health detection data is relatively low due to a complex tissue structure of a to-be-monitored part of a user.

To achieve the foregoing objective, this application uses the following technical solutions. According to one aspect of this application, a detection apparatus is provided. The detection apparatus includes at least three first light sources, at least three first photodetectors, and at least two second light sources. At least one first photodetector is disposed between two adjacent first light sources, and a detection region is enclosed by the at least three first light sources and the at least three first photodetectors. In addition, the second light sources are disposed in the detection region. In a same distribution region, there is a first distance H1 between the first light source and at least one first photodetector. There is a second distance H2 between the first light source and at least one first photodetector outside the distribution region. In a same distribution region, there is a third distance H3 between the second light source and at least one first photodetector. There is a fourth distance H4 between the second light source and at least one first photodetector outside the distribution region. Any two of the first distance H1, the second distance H2, the third distance H3, and the fourth distance H4 are different. On this basis, the detection apparatus has at least two distribution regions, and the first light sources, the second light sources, and the first photodetectors are distributed in the distribution regions. The first light sources configured to enclose the detection region may be referred to as outer-ring first light sources, and the second light sources located in the detection region may be referred to as inner-ring light sources.

In conclusion, at least one first photodetector is disposed between two adjacent first light sources, and the detection region is enclosed by the at least three first light sources and the at least three first photodetectors. When the detection apparatus is in contact with skin of a user, the detection region determines a region range of the skin of the user that can be detected by the detection apparatus. In addition, the at least two second light sources are disposed in the detection region, the detection apparatus has at least two distribution regions, and the first light sources, the second light sources, and the first photodetectors are distributed in the distribution regions. In this case, one light path may be formed between one first light source and one first photodetector, so that the first light source and the first photodetector in the light path may form one PPG module. Similarly, one light path may also be formed between one second light source and one first photodetector, so that the second light source and the first photodetector in the light path may form one PPG module. The PPG module can detect pulse data of the user. In this way, a quantity of light paths and a quantity of PPG modules corresponding to the light paths in the detection region may be increased by setting the distribution regions, so that when the skin of the user is in contact with the detection region, results of detecting the pulse data of the user by more PPG modules may be obtained, thereby alleviating a problem that detection data accuracy is reduced due to interference to detection data in a partial region in the detection region. On this basis, a distance between a light source and a photodetector may determine an effective depth that is in the skin and at which light emitted by the light source arrives (at the effective depth, a proportion of PPG signals is relatively high). Therefore, in the same distribution region, there is the first distance H1 between the first light source and the at least one first photodetector. There is the second distance H2 between the first light source and the at least one first photodetector outside the distribution region. In the same distribution region, there is the third distance H3 between the second light source and the at least one first photodetector. There is the fourth distance H4 between the second light source and the at least one first photodetector outside the distribution region. In this case, the first light source may have the first distance H1 and the second distance H2 from at least two of all first photodetectors, and the second light source may have the third distance H3 and the fourth distance H4 from at least two of all first photodetectors. Any two of the foregoing four distances are different. Therefore, the detection apparatus may have light paths with at least four distances. A PPG module corresponding to each light path may obtain PPG data of one skin depth, so that the detection apparatus can obtain PPG data of at least four skin depths. In this way, based on detection results of the detection apparatus, an electronic device may not only compare and analyze PPG data obtained from different light paths in the detection region, but also compare and analyze PPG data with different PIs, so that finally output PPG data has relatively high accuracy.

In an optional implementation, 0 mm < H1 < 4 mm, and in the same distribution region, a light path formed between the first light source and the first photodetector is a light path with a short distance range (less than 4 mm). 4 mm ≤ H3 < 7 mm, and in the same distribution region, a light path formed between the second light source and the first photodetector is a light path with a medium distance range (4 mm to 7 mm). 7 mm ≤ H4 ≤ 10 mm, and a light path formed between the second light source and the at least one first photodetector outside the distribution region is a light path with a long distance range (7 mm to 10 mm). H2 > 10 mm, and a light path formed between the first light source and the at least one first photodetector outside the distribution region is a light path with an ultra-long distance range (greater than 10 mm). Alternatively, 0 mm < H3 < 4 mm, 4 mm ≤ H1 < 7 mm, 7 mm ≤ H4 ≤ 10 mm, and H2 > 10 mm. Setting manners and technical effects of the distances are the same as those described above. Details are not described herein again.

In an optional implementation, two first light sources, and one second light source and at least one first photodetector that are located between the two first light sources are distributed in the distribution region. In this way, in the same distribution region, not only the two adjacent first light sources may separately form a light path with the first photodetector, but also the second light source between the two first light sources may form a light path with the first photodetector, so that a quantity of light paths between the two adjacent first light sources may be increased. Therefore, in the entire detection region, an area covered by light paths is larger, and the detection apparatus obtains a larger quantity of PPGs from different light paths, so that impact of interference caused by a relatively large proportion of DC signals in a partial region on a detection result may be reduced, thereby helping improve the detection accuracy.

In an optional implementation, the detection apparatus includes three first light sources and six first photodetectors, two first photodetectors are disposed between two adjacent first light sources, and two first photodetectors are distributed in the distribution region. Technical effects of the first light source and the first photodetector are the same as those described above. Details are not described herein again.

In an optional implementation, in a same distribution region, the first light source has the first distance H1 and a fifth distance H5 respectively from two first photodetectors, and the first distance H1 and the fifth distance H5 are different. For example, H1 < H5, and 4 mm ≤ H5 < 7 mm, or 7 mm ≤ H5 ≤ 10 mm. In the same distribution region, a light path formed between the first light source and one of the first photodetectors is a light path with the short distance range, and a light path formed between the first light source and the other first photodetector is a light path with the medium distance range. In a same distribution region, the second light source has the third distance H3 and a sixth distance H6 respectively from two first photodetectors. For example, 4 mm ≤ H6 < 7 mm, and in the same distribution region, a light path formed between the second light source and any first photodetector is a light path with the medium distance range. In this way, the detection apparatus can obtain PPG data of six depths. Technical effects of PPG data of a plurality of depths are the same as those described above. Details are not described herein again. In an optional implementation, the first distances H1 in different distribution regions are the same, the second distances H2 in different distribution regions are the same, the third distances H3 in different distribution regions are the same, and the fourth distances H4 in different distribution regions are the same. In this way, distribution of the first light sources, the second light sources, and the first photodetectors in the detection apparatus may be symmetric, to improve appearance quality of the electronic device.

In an optional implementation, the detection apparatus includes three first light sources and three first photodetectors, one first photodetector is disposed between two adjacent first light sources, and one first photodetecting is distributed in the distribution region. A manner of disposing the first light source and the first photodetector is the same as that described above. Details are not described herein again.

In an optional implementation, the detection apparatus has three distribution regions, the detection apparatus includes three second light sources, one second light source is located in one distribution region, and two adjacent distribution regions share a same first light source. Technical effects of the second light source, the first light source, and the distribution region are the same as those described above. Details are not described herein again.

In an optional implementation, the detection apparatus has two distribution regions, the detection apparatus includes two second light sources, one second light source is located in one distribution region, and two adjacent distribution regions share a same first light source. Technical effects of the second light source, the first light source, and the distribution region are the same as those described above. Details are not described herein again.

In an optional implementation, the detection apparatus further includes three third light sources, and one third light source is disposed between two adjacent first photodetectors. In this way, each third light source may form two light paths respectively with two first photodetectors adjacent to the third light source, so that a quantity of light paths between the two adjacent first photodetectors may be increased.

In an optional implementation, the detection apparatus includes four first light sources, four first photodetectors, and two second light sources, one first photodetector is disposed between two adjacent first light sources, and one first photodetector is distributed in the distribution region. The detection apparatus has two distribution regions, and one second light source is located in one distribution region. Technical effects of the first light source, the first photodetector, and the distribution region are the same as those described above. Details are not described herein again. In an optional implementation, the detection apparatus includes four first light sources and four first photodetectors, and one first photodetector is disposed between two adjacent first light sources. The detection apparatus further includes four second photodetectors, and one second photodetector is disposed between two adjacent first photodetectors. Two second photodetectors and one first photodetector are distributed in the distribution region. In addition, the detection apparatus has two distribution regions, the detection apparatus includes two second light sources, and one second light source is located in one distribution region. In this way, in the same distribution region, either of the first light source and the second light source may not only form a light path with the first photodetector, but also form a light path with the second photodetector, thereby increasing a quantity of light paths. In addition, a manner of setting a distance between the light source and each photodetector may be similarly obtained. Details are not described herein again.

In an optional implementation, the detection apparatus further includes four second photodetectors, and the second photodetectors are disposed in the detection region. The detection apparatus includes four first light sources and four first photodetectors, and one first photodetector is disposed between two adjacent first light sources. Two first photodetectors, and one second photodetector, one second light source, and one first light source that are located between the two first photodetectors are distributed in the distribution region, and the second photodetector is located between the first light source and the second light source. The detection apparatus has two distribution regions, and one second light source is located in one distribution region. A manner of setting a distance between the light source and each photodetector may be similarly obtained. Details are not described herein again.

In an optional implementation, either of the first light source and the second light source includes a first light emitting device and a second light emitting device, the first light emitting device emits red light, and the second light emitting device emits infrared light. The red light is mainly absorbed by deoxygenated hemoglobin in blood, and the infrared light is mainly absorbed by oxygenated hemoglobin in the blood. Therefore, a processor of the electronic device may calculate blood oxygen saturation by measuring a ratio of the deoxygenated hemoglobin to the oxygenated hemoglobin in the blood by using an absorption difference that is between the red light and the infrared light and that is detected by the detection apparatus, to obtain blood oxygen data.

In an optional implementation, either of the first light source and the second light source further includes a third light emitting device, and the third light emitting device emits green light. Because human blood has a higher absorption rate for green light, when the first light emitting device emits green light, heart rate data detected by the detection apparatus may be more accurate.

In an optional implementation, the detection apparatus further includes a transparent cover, an optical film layer, an ink layer, and a light shielding member. The transparent cover covers the first light source, the first photodetector, and the second light source. The transparent cover may be made of a transparent material whose light transmittance reaches 80% or higher, for example, glass, transparent resin, or sapphire. The optical film layer is disposed on a side that is of the transparent cover and that faces the first light source. The optical film layer may transmit light emitted by a light source and light reflected or scattered by the skin, and may further hide a component that is in the electronic device and that is covered by the transparent cover, to prevent the user from clearly seeing an internal structure of the detection apparatus. The ink layer is disposed between the optical film layer and the transparent cover, and a first transparent hole, a second transparent hole, and a third transparent hole are provided on the ink layer. The first transparent hole exposes a light emitting surface of the first light source, the second transparent hole exposes a light emitting surface of the second light source, and the third transparent hole exposes a light receiving surface of the first photodetector. The ink layer may hide components in the detection apparatus other than the first light source, the second light source, and the first photodetector, and expose the first light source, the second light source, and the first photodetector respectively through the first transparent hole, the second transparent hole, and the third transparent hole, to form the light paths. The light shielding member is disposed on a side that is of the optical film layer and that faces away from the transparent cover, and the light shielding member is located between any two of the first light source, the second light source, and the first photodetector. The light shielding member can prevent light crosstalk between the first light source and the second light source, or prevent light emitted by the first light source (or the second light source) from being directly received by the first photodetector without being absorbed and scattered by the skin.

According to one aspect of this application, an electronic device is provided. The electronic device includes a circuit board and any one of the foregoing detection apparatuses, and the detection apparatus is disposed on the circuit board. The electronic device has same technical effects as the detection apparatus provided in the foregoing embodiment. Details are not described herein again.

### BRIEF DESCRIPTION OF DRAWINGS

FIG. 1A is a diagram of a structure of an electronic device according to an embodiment of this application;
FIG. 1B is an exploded diagram of the structure of the electronic device in FIG. 1A;
FIG. 2A is a diagram of an arrangement position that is of a detection apparatus in FIG. 1B and that is in an electronic device;
FIG. 2B is a diagram of an arrangement position that is of a detection apparatus in FIG. 1B and that is in another electronic device;
FIG. 2C is a diagram of an arrangement position that is of a detection apparatus in FIG. 1B and that is in another electronic device;
FIG. 3 is a diagram of wearing an electronic device according to an embodiment of this application;
FIG. 4 is a diagram of a structure of a detection apparatus according to an embodiment of this application;
FIG. 5A is a diagram of detection by using an oximeter according to a related technology;
FIG. 5B is a diagram of a detection principle of the oximeter in FIG. 5A;
FIG. 6 is a diagram of a cross-sectional structure of human skin according to an embodiment of this application;
FIG. 7 is a diagram of a PPG wave generation principle according to an embodiment of this application;
FIG. 8A is a diagram of PPG waves obtained from different parts of a human body according to an embodiment of this application;
FIG. 8B is a diagram of a detection principle of a detection apparatus according to an embodiment of this application;
FIG. 9A is a diagram of a structure of a detection apparatus according to an embodiment of this application;
FIG. 9B is a diagram of distribution region division of the detection apparatus shown in FIG. 9A;
FIG. 9C is a diagram of other distribution region division of the detection apparatus shown in FIG. 9A;
FIG. 10 is a diagram of a structure of another detection apparatus according to an embodiment of this application;
FIG. 11A is a diagram of a light path of the detection apparatus shown in FIG. 9A;
FIG. 11B is a diagram of a light path of a detection apparatus according to an embodiment of this application;
FIG. 11C is a diagram of another light path of a detection apparatus according to an embodiment of this application;
FIG. 12 is a diagram of a detection principle of another detection apparatus according to an embodiment of this application;
FIG. 13 is a diagram of light paths with a plurality of different distances of the detection apparatus shown in FIG. 9A;
FIG. 14 is a diagram of a display state of an electronic device according to an embodiment of this application;
FIG. 15 is a diagram of a structure of a first light source in FIG. 9A;
FIG. 16 is a diagram of a structure of another detection apparatus according to an embodiment of this application;
FIG. 17A is a diagram of distribution region division of the detection apparatus shown in FIG. 16;
FIG. 17B is a diagram of a light path of the detection apparatus shown in FIG. 16;
FIG. 17C is a diagram of light paths with a plurality of different distances of the detection apparatus shown in FIG. 16;
FIG. 17D is another diagram of light paths with a plurality of different distances of the detection apparatus shown in FIG. 16;
FIG. 18 is a diagram of signal proportions at different skin depths according to an embodiment of this application;
FIG. 19 is a diagram of a structure of another detection apparatus according to an embodiment of this application;
FIG. 20 is a diagram of a structure of another detection apparatus according to an embodiment of this application;
FIG. 21 is a diagram of light paths with a plurality of different distances of the detection apparatus shown in FIG. 20;
FIG. 22 is a diagram of a structure of another detection apparatus according to an embodiment of this application; and
FIG. 23 is a diagram of a structure of another detection apparatus according to an embodiment of this application.

### Reference numerals:

01: electronic device; 10: display; 11: middle frame; 12: rear cover; 13: circuit board; 20: detection apparatus; 100: skin; 02: oximeter; 101: horny layer; 102: epidermis layer; 103: dermis layer; 104: subcutaneous tissue; 1021: capillary; 1031: small artery; 1041: large artery; 201: first light source; 202: second light source; 211: first photodetector; 200: detection region; 300: distribution region; 301: optical film layer; 302: transparent cover; 303: light shielding member; 304: ink layer; 3041: first transparent hole; 3042: second transparent hole; 3043: third transparent hole; 203: third light source; 221: second photodetector.

### DESCRIPTION OF EMBODIMENTS

The following describes technical solutions in embodiments of this application with reference to accompanying drawings in embodiments of this application. It is clear that the described embodiments are merely a part rather than all of embodiments of this application.

The terms such as "first" and "second" below are merely for convenience of description, and are not to be construed as indicating or implying relative importance or implicitly indicating a quantity of indicated technical features. Therefore, a feature limited by "first", "second", or the like may explicitly or implicitly include one or more features. In the descriptions of this application, unless otherwise stated, "a plurality of" means two or more.

In this application, unless otherwise expressly specified and limited, the term "connection" should be understood in a broad sense. For example, "connection" may be a fixed mechanical connection, or may be a detachable mechanical connection or an integration. Alternatively, "connection" may be a direct connection, or may be an indirect connection via an intermediate medium.

In addition, unless otherwise expressly specified and limited, the term "electrical connection" should be understood in a broad sense. For example, "electrical connection" may be a direct electrical connection, for example, two components are physically in contact and electrically connected; or may be understood as that in a line structure, different components are electrically connected through a physical line that can transmit an electrical signal, such as a printed circuit board (printed circuit board, PCB) copper foil or a conducting wire, to transmit an electrical signal. Alternatively, "electrical connection" may be an indirect electrical connection between two components via an intermediate medium. Alternatively, "electrical connection" may be an electrical connection between two components in a separated or non-contact manner. For example, two components are electrically connected in a capacitive coupling manner, to transmit an electrical signal.

In the accompanying drawings of embodiments of this application, an assembly is indicated by using a leader line with an arrow, a component is indicated by using only a leader line, and a hollow-out structure such as an opening or a hole is indicated by using a leader line with a wavy line at an end.

An embodiment of this application provides an electronic device. The electronic device may be applied to various communication systems or communication protocols, for example, a Bluetooth (Bluetooth, BT) communication technology, a global positioning system (global positioning system, GPS) communication technology, a global system for mobile communications (global system for mobile communications, GSM) communication technology, a wireless fidelity (wireless fidelity, Wi-Fi) communication technology, a wideband code division multiple access (wideband code division multiple access, WCDMA) communication technology, long term evolution (long term evolution, LTE), a 5G communication technology, and another future communication technology.

The electronic device in this embodiment of this application may be a smart wearable device, for example, a human health detection wearable device that can detect a health sign parameter (for example, a heart rate, blood oxygen, blood pressure, or sleep) of a user. For example, the smart wearable device may be a smartwatch, a smart band, smart glasses, a smart helmet, or a smart headset. Alternatively, the electronic device may be a mobile phone (mobile phone), a tablet computer (pad), a notebook computer, a smart home device, a virtual reality (virtual reality, VR) electronic device, an augmented reality (augmented reality, AR) electronic device, or the like. Alternatively, the electronic device may be a handheld device that has a wireless communication function, a computing device, another processing device connected to a wireless modem, a vehicle-mounted device, an electronic device in a 5G network, an electronic device in a future evolved public land mobile network (public land mobile network, PLMN), or the like. This is not limited in this embodiment of this application.

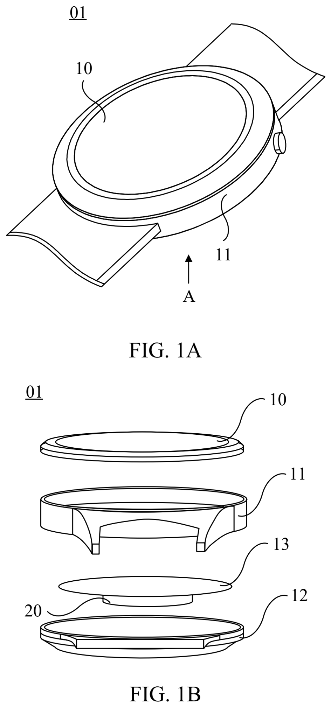

For ease of description, an example in which an electronic device 01 shown in FIG. 1A is a smartwatch that can perform health detection is used below for description. For example, the electronic device 01 may include a display (display) 10, a middle frame 11, and a rear cover (rear cover) 12 shown in FIG. 1B. Still as shown in FIG. 1B, the display 10 may be disposed in the middle frame 11, a display surface of the display 10 is located on a side that faces away from the rear cover 12, and the middle frame 11 may be disposed around a periphery of the display 10. The display 10 may be a liquid crystal display (liquid crystal display, LCD), an organic light emitting diode (organic light emitting diode, OLED) display, or a micro (micro or mini) light emitting diode (light emitting diode, LED) display. A shape of the display 10 is not limited in this application. For example, the display surface of the display 10 may be a circle or a rectangle. Contour shapes of the middle frame 11 and the rear cover 12 may match a contour shape of the display 10. For ease of description, an example in which the contour shapes of the display 10, the middle frame 11, and the rear cover 12 are circular is used below for description.

On this basis, the electronic device 01 may further include components such as a circuit board 13 (as shown in FIG. 1B), a battery, a processor, a sensor, a microphone, and a speaker. In some embodiments of this application, components such as the circuit board, the battery, the processor, the sensor, the microphone, and the speaker may be disposed between the display 10 and the rear cover 12. The middle frame 11 may support the entire electronic device 01.

For example, the processor may include one or more processing units. For example, the processor may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, and/or a neural-network processing unit (neural-network processing unit, NPU). Different processing units may be independent components, or may be integrated into one or more processors.

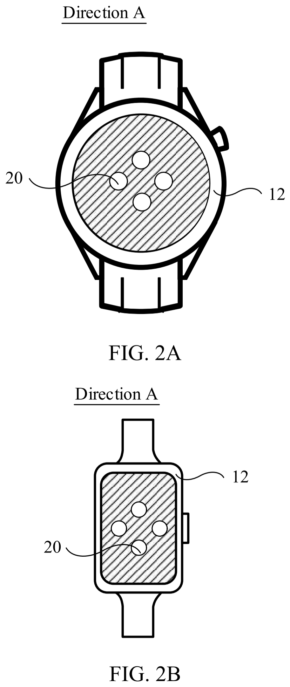

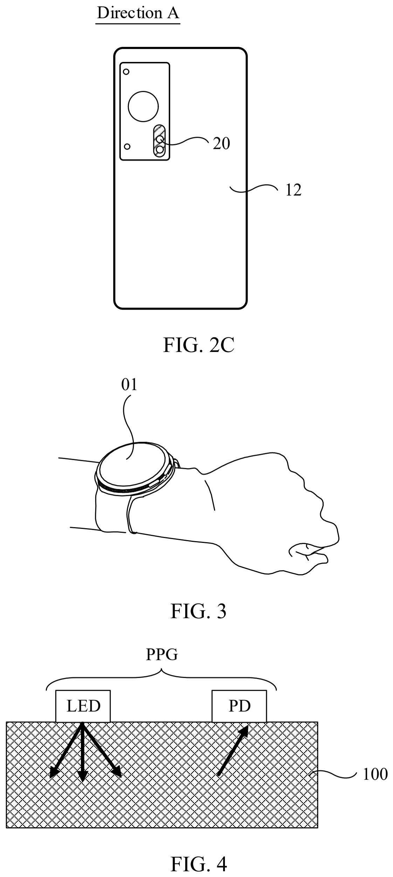

In addition, to detect a health sign parameter, for example, data such as a heart rate and blood oxygen, the electronic device 01 may further include a detection apparatus 20 shown in FIG. 1B. The detection apparatus 20 may be located in accommodating space enclosed by the display 10, the middle frame 11, and the rear cover 12. The detection apparatus 20 may be disposed on the circuit board 13, and is electrically connected to the circuit board 13. Based on this, as shown in FIG. 2A (a top view obtained along a direction A in FIG. 1A), the rear cover 12 may expose a part of the detection apparatus 20. In FIG. 2A, an example in which the electronic device 01 is a smartwatch is used for description. When the electronic device 01 is a band shown in FIG. 2B (a top view obtained along the direction A in FIG. 1A) or a mobile phone shown in FIG. 2C (a top view obtained along the direction A in FIG. 1A), the rear cover 12 of the electronic device 01 may also expose a part of the detection apparatus 20.

FIG. 2A, FIG. 2B, and FIG. 2C are merely descriptions of examples in which the rear cover 12 exposes a part of the detection device 20, and do not constitute limitations on a shape design of the rear cover 12 and a structure of the detection device 20. In some other embodiments of this application, the electronic device 01 may alternatively be a headset or a head-mounted device. When the detection apparatus 20 performs health detection on a user, the rear cover 12 of the electronic device 01 may be in contact with skin of the user. For ease of description, an example in which the electronic device 01 is the smartwatch shown in FIG. 2A is used below for description. Based on this, when the electronic device 01 is the smartwatch, as shown in FIG. 3, the user may wear the electronic device 01 on a left hand (or a right hand), and the rear cover 12 (as shown in FIG. 2A) of the electronic device 01 may be in contact with a wrist of the user, so that the detection apparatus 20 can obtain blood oxygen and heart rate data of the user. For example, the detection apparatus 20 may include at least one photoplethysmograph (photoplethysmograph, PPG) module shown in FIG. 4.

A specific working principle of the PPG module may be that human blood circulation is driven by systole and diastole of a beating heart. During systole of the heart, blood flows around a body, hyperemia occurs in a to-be-monitored part, and a blood volume increases. During diastole of the heart, blood at the to-be-monitored part flows back to the heart, and the blood volume decreases. In this way, a periodic signal is generated, which may also be referred to as an alternating current (alternating current, AC) signal. The AC signal may include a systolic phase (systolic phase) peak value and a diastolic phase (diastolic phase) peak value, and a heart rate (heart rate, HR) may be determined by using the AC signal. In addition, blood oxygen content in blood is related to hemoglobin content.

Based on this, the PPG module shown in FIG. 4 may include a light source such as an LED and a photodetector (photodetector, PD). The LED and the PD are disposed on a same side of skin 100 of the user, and the PPG module is a reflective PPG module. The human skin 100 absorbs and scatters visible light and infrared light. After light emitted by the LED shown in FIG. 4 is irradiated onto the skin 100, a blood flow status (for example, a blood volume change caused by a heartbeat) in the skin 100 and hemoglobin content in blood affect light absorption, and further affect light scattering by the skin 100. Light scattered by the skin 100 is obtained by the PD, so that a regularity of time-dependent variations in light intensity associated with a blood volume in the human body, that is, a photoplethysmogram, may be obtained. A blood flow characteristic and hemoglobin content may be reflected by the photoplethysmogram, so that data such as a heart rate and blood oxygen may be obtained.

The foregoing description is provided by using an example in which the light source in the PPG module is an LED. In some other embodiments of this application, the light source may alternatively be a self-luminous device such as an OLED or a quantum dot light emitting diode (quantum dot light emitting diode, QLED). A structure of the light source is not limited in this application.

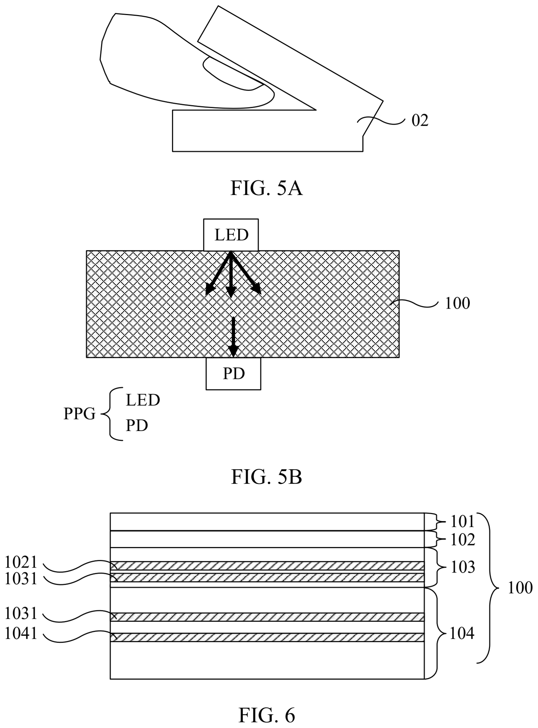

In a related technology, as shown in FIG. 5A, the user may clamp a finger into a finger-clip oximeter 02 to detect a fingertip of the user by using the finger-clip oximeter 02, to obtain blood oxygen and heart rate data. In this case, as shown in FIG. 5B, the LED and the PD in the PPG module may be respectively disposed on two sides of the skin 100 of the user. The PPG module is a transmissive PPG module. However, in the detection manner of using the finger-clip oximeter 02, the fingertip of the user cannot normally move and has a squeezing sensation, and it is difficult to implement real-time detection. Therefore, the electronic device 01 provided in this embodiment of this application uses the reflective PPG module shown in FIG. 4, and an example in which the detection apparatus 20 in the electronic device 01 tests the wrist of the user is used for description. Based on this, as shown in FIG. 6, the human skin 100 from outside to inside may sequentially include a horny layer 101, an epidermis layer 102, a dermis layer 103, and a subcutaneous tissue 104. The epidermis layer 102 includes a granular layer, a spinous layer, a basal layer (not shown in the figure), and the like. The dermis layer 103 may include a papillary dermis layer, an upper vascular plexus, a reticular dermis layer, a lower vascular plexus (not shown in the figure), and the like. Capillaries 1021, arterioles (not shown in the figure), and most of small arteries 1031 are distributed in the dermis layer 103. The subcutaneous tissue 104 may include an adipose layer, a fascia (not shown in the figure), and the like. A small part of small arteries 1031 and a large artery 1041 are distributed in the subcutaneous tissue 104. FIG. 6 is merely intended to indicate that the capillaries 1021 and the small arteries 1031 are distributed in the dermis layer 102, and the small part of small arteries 1031 and the large artery 1041 are distributed in the subcutaneous tissue 104, and does not constitute a limitation on a relative position relationship between the capillaries 1021, the small arteries 1031, and the large artery 1041.

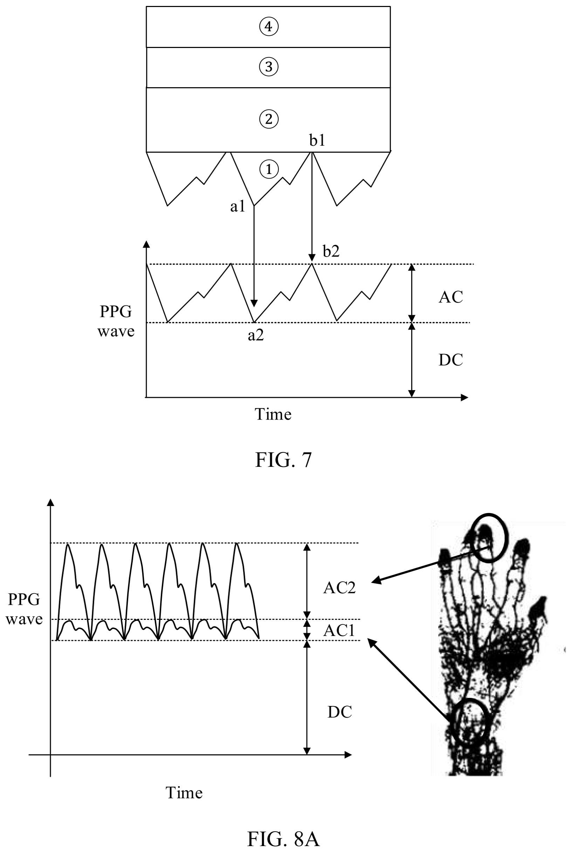

In this case, in data obtained by the PPG module, as shown in FIG. 7, a part that can generate the AC signal is a pulsatile component of artery blood (pulsatile component of artery blood) ① of the large artery 1041 (as shown in FIG. 6). When the pulsatile component ① of the artery blood is located at a peak position a1, a blood volume in the large artery 1041 (as shown in FIG. 6) reaches a maximum. Therefore, the artery blood has a highest absorption rate for the light emitted by the LED in the PPG module. In this case, the PD in the PPG receives a smallest quantity of optical signals, which correspond to a trough position a2 of the AC signal. In addition, when the pulsatile component ① of the artery blood is located at a trough position b1, the blood volume in the large artery 1041 (as shown in FIG. 6) reaches a minimum. Therefore, the artery blood has a lowest absorption rate for the light emitted by the LED in the PPG module. In this case, the PD in the PPG receives a largest quantity of optical signals, which correspond to a peak position b2 of the AC signal. A waveform of the AC signal periodically changes over time.

In addition, a non-pulsatile component of artery blood (non-pulsatile component of artery blood) ②, venous blood (venous blood) ③, and other tissues ④ (for example, a bone, a muscle, a pigment, and hair of the human body) that are shown in FIG. 7 have basically unchanged absorption rates for the light emitted by the LED in the PPG module. Therefore, optical signals received by the PD in the PPG from the foregoing three parts (②, ③, and ④ in FIG. 7) basically remain unchanged. Therefore, a PPG detection result not only includes the AC signal, but also includes direct current (direct current, DC) signals generated by the foregoing three parts (②, ③, and ④ in FIG. 7). Magnitudes of the DC signals do not change over time.

Based on this, as shown in FIG. 8A, vascular distribution density of a wrist part of the human body is less than vascular distribution density of a fingertip part, and a human body structure of the wrist part is more complex than a human body structure of the fingertip part. For example, the wrist part not only includes skin, but also includes parts that can generate DC signals, such as the bone, the muscle, the pigment, and the hair. Therefore, an amplitude (or signal strength) of an alternating current signal component AC1 obtained by the PPG module by detecting the wrist part is less than an amplitude (or signal strength) of an alternating current signal component AC2 obtained by the PPG module by detecting the fingertip part.

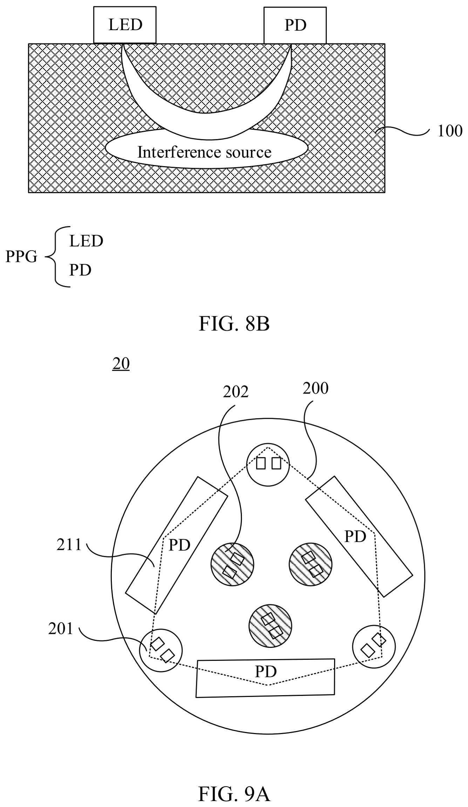

Detection accuracy of the detection apparatus 20 with the foregoing PPG module is affected by a perfusion index (perfusion index, PI). A larger PI leads to higher detection accuracy of the detection apparatus 20, and a smaller PI leads to lower detection accuracy of the detection apparatus 20. The PI may be a ratio of an AC signal to a DC signal. Therefore, it can be learned from FIG. 8A that a PI value obtained by the detection apparatus 20 by detecting the wrist part is less than a PI value obtained by the detection apparatus 20 by detecting the fingertip part. In addition, as shown in FIG. 8B, a part that is of the skin 100 of the user and that is used to generate a DC signal serves as an interference source, and causes interference to a signal detected by the PPG module, thereby reducing a signal-to-noise ratio (signal-to-noise ratio, SNR) of the detection apparatus 20. In addition, the detection accuracy of the detection apparatus 20 is also affected by other noise, for example, noise caused by factors such as electronic circuit interference and a device status deviation. After the light emitted by the LED enters the skin, a "banana-shaped" light path shown in FIG. 8B is formed, and the light is received by the PD through the foregoing light path, so that the PPG module can detect data such as blood oxygen and a heart rate of the user. Based on this, to improve the detection accuracy and the signal-to-noise ratio of the detection apparatus 20, as shown in FIG. 9A, the detection apparatus 20 provided in this embodiment of this application may include at least three first light sources 201, at least three first photodetectors 211, and at least two second light sources 202. In FIG. 9A, an example in which the detection apparatus includes three first light sources 201, three first photodetectors 211, and three second light sources 202 is used for description.

Based on this, still as shown in FIG. 9A, at least one first photodetector 211 is disposed between two adjacent first light sources 201. A detection region 200 may be enclosed by all first light sources 201 (that is, the at least three first light sources 201) and all first photodetectors 211 (that is, the at least three first photodetectors 211) of the detection apparatus 20. All second light sources 202 (that is, the at least two second light sources 202) of the detection apparatus 20 may be disposed in the detection region 200. The first light sources 201 configured to enclose the detection region 200 may be referred to as outer-ring first light sources, and the second light sources 202 located in the detection region 200 may be referred to as inner-ring light sources.

The detection region 200 is a range of skin covered by the detection region 200 when the user brings the electronic device 01 into contact with the skin, for example, when the user wears the electronic device 01 on the wrist, and the rear cover 12 of the electronic device 01 comes into contact with skin of the wrist of the user. The range of the skin covered by the detection region 200 may be a region range of skin of the user that can be detected by the detection apparatus 20 in the electronic device 01.

For ease of description, in the accompanying drawings, the first light sources 201 are represented by circles filled with no pattern, the second light sources 202 are represented by circles filled with oblique lines, and the first photodetectors 211 are represented by squares filled with no pattern. In the accompanying drawings, a shape of a component does not constitute a limitation on an actual shape of the component.

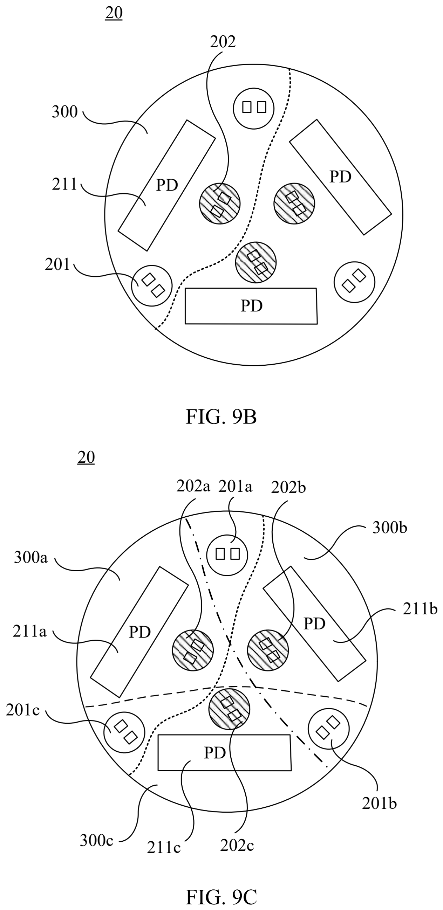

On this basis, as shown in FIG. 9B, the detection apparatus 20 may have a distribution region 300 (a region enclosed by a dashed line and an edge of the detection apparatus 20 that intersect in FIG. 9B). A first light source 201, a second light source 202, and a first photodetector 211 are distributed in the distribution region 300. For example, two first light sources 201, one second light source 202, and one first photodetector 211 may be distributed in the distribution region 300 shown in FIG. 9B.

In FIG. 9B, an example in which the detection apparatus 20 has three second light sources 202 is used for description. Based on this, as shown in FIG. 9C, the detection apparatus 20 may include three distribution regions: a first distribution region 300a, a first distribution region 300b, and a first distribution region 300c. One second light source 202 is disposed in each of the first distribution region 300a, the first distribution region 300b, and the first distribution region 300c. The detection apparatus 20 may include three first light sources (for example, a first light source 201a, a first light source 201b, and a first light source 201c), three second light sources (for example, a second light source 202a, a second light source 202b, and a second light source 202c), and three first photodetectors (for example, a first photodetector 211a, a first photodetector 211b, and a first photodetector 211c).

In addition, in any one of the first distribution region 300a, the first distribution region 300b, and the first distribution region 300c, there are two first light sources and one first photodetector. Two adjacent distribution regions may share a same first light source 201. For example, the first distribution region 300a and the first distribution region 300b share one first light source 201a, the first distribution region 300b and the first distribution region 300c share one first light source 201b, and the first distribution region 300c and the first distribution region 300a share one first light source 201c.

In this case, in a same distribution region (for example, the distribution region 300a), when the first photodetector 211a is disposed between two adjacent first light sources, for example, the first light source 201a and the first light source 201c, the second light source 202a may be distributed near a position of the first photodetector 211a by disposing the second light source 202a between the two adjacent first light sources. In this way, in the same distribution region, not only the first light source 201a and the first light source 201c may separately form a light path with the first photodetector 211a, but also the second light source 202a between the first light source 201a and the first light source 201c may form a light path with the first photodetector 211a, so that a quantity of light paths between the two adjacent first light sources (for example, the first light source 201a and the first light source 201c) may be increased. Therefore, in the entire detection region 200 (as shown in FIG. 9A), an area covered by light paths is larger, and the detection apparatus 20 obtains a larger quantity of PPGs from different light paths, so that impact of interference caused by a relatively large proportion of DC signals in a partial region on a detection result may be reduced, thereby helping improve the detection accuracy.

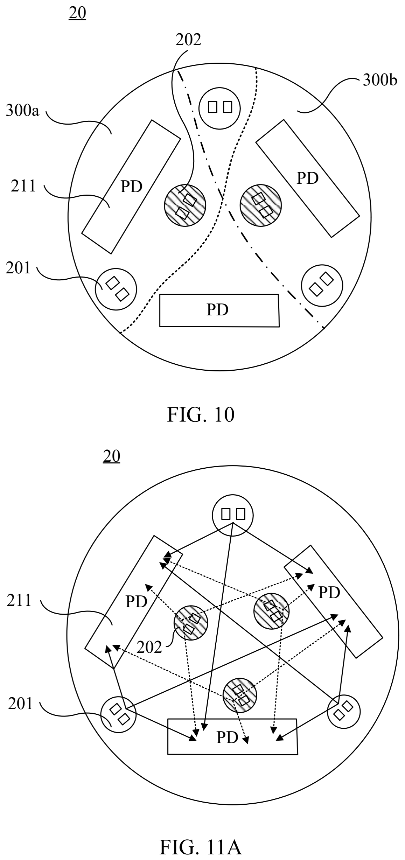

The foregoing description is provided by using an example in which the detection apparatus 20 shown in FIG. 9C has three second light sources 202. In some other embodiments of this application, as shown in FIG. 10, the detection apparatus 20 may have two second light sources 202. In this case, the detection apparatus 20 may have two distribution regions: a first distribution region 300a and a first distribution region 300b. One second light source 202 is disposed in each of the first distribution region 300a and the first distribution region 300b. In addition, as described above, two adjacent distribution regions (for example, the first distribution region 300a and the second distribution region 300b) share a same first light source 201.

The foregoing is an example of setting a quantity of distribution regions 300 based on different quantities of second light sources 202, and does not constitute a limitation on the quantity of second light sources 202 and the quantity of distribution regions 300, provided that the distribution region 300 has a first light source 201, a second light source 202, and a first photodetector 211. For ease of description, the following uses the three second light sources shown in FIG. 9C as an example for description.

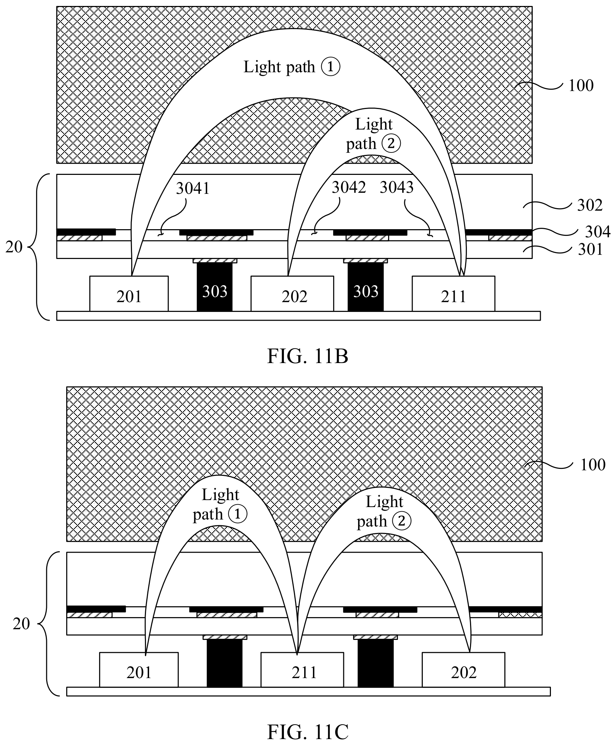

In this case, as shown in FIG. 11A, in the detection apparatus 20, one light path (represented by a solid arrow in FIG. 11A) may be formed between one first light source 201 and one first photodetector 211. For example, as shown in FIG. 11B, light emitted by a first light source 201 is incident on the skin 100, and after the light is absorbed and scattered by the skin 100, a part of scattered light may be incident on a first photodetector 211, thereby forming a light path ①. In this way, the first light source 201 and the first photodetector 211 that form the light path ① may form one PPG module, and the PPG module can detect pulse data of the user.

As shown in FIG. 11B, the detection apparatus 20 may further include an optical film layer 301, a transparent cover 302, a light shielding member 303, and an ink layer 304. The transparent cover 302 may serve as at least a part of the rear cover 12 in FIG. 1B. The transparent cover 302 may cover the first light source 201, the first photodetector 211, and the second light source 202. The transparent cover 302 may be made of a transparent material whose light transmittance reaches 80% or higher, for example, glass, transparent resin, or sapphire. In addition, the optical film layer 301 may be disposed on a side that is of the transparent cover 302 and that faces the first light source 201. The optical film layer 301 may be a Fresnel film layer. The optical film layer 301 may transmit light emitted by a light source and light reflected or scattered by the skin, and may further hide a component that is in the electronic device 01 and that is covered by the transparent cover 302, to prevent the user from clearly seeing an internal structure of the detection apparatus 20.

In addition, still as shown in FIG. 11B, the ink layer 304 may be disposed between the optical film layer 301 and the transparent cover 302, and first transparent holes 3041, second transparent holes 3042, and third transparent holes 3043 are provided on the ink layer 304. The first transparent holes 3041 may expose light emitting surfaces of the first light sources 201, the second transparent holes 3042 may expose light emitting surfaces of the second light sources 202, and the third transparent holes 3043 may expose light receiving surfaces of the first photodetectors 211. In this way, the ink layer 304 may hide components in the detection apparatus 20 other than the first light sources 201, the second light sources 202, and the first photodetectors 211, and expose the first light sources 201, the second light sources 202, and the first photodetectors 211 respectively through the first transparent holes 3041, the second transparent holes 3042, and the third transparent holes 3043, to form the light path ① and a light path ②.

On this basis, to prevent light crosstalk between a first light source 201 and a second light source 202, or to prevent light emitted by a first light source 201 (or a second light source 202) from being directly received by a first photodetector 211 without being absorbed and scattered by the skin 100, the detection apparatus 20 may include the light shielding member 303. The light shielding member 303 may be disposed on a side that is of the optical film layer 301 and that faces away from the transparent cover 302, and the light shielding member 303 may be located between any two of the first light source 201, the second light source 202, and the first photodetector 211, to isolate light. A transparent adhesive layer (represented by a pattern filled with oblique lines in the figure) may be used for bonding between the light shielding member 303 and the optical film layer 301, and between the optical film layer 301 and the ink layer 304.

Similarly, still as shown in FIG. 11A, one light path (represented by a dashed arrow in FIG. 11A) may also be formed between one second light source 202 and one first photodetector 211. For example, as shown in FIG. 11B, light emitted by a second light source 202 is incident on the skin 100, and after the light is absorbed and scattered by the skin 100, a part of scattered light may be incident on a first photodetector 211, thereby forming a light path ②. In this way, the second light source 202 and the first photodetector 211 that form the light path ② may form one PPG module. Based on this, a first light source 201, a second light source 202, and a first photodetector 211 are disposed in each distribution region 300 (as shown in FIG. 9B) of the detection setting 20 provided in this embodiment of this application, and the detection apparatus 20 may have at least two distribution regions. Therefore, a quantity of light paths and a quantity of PPG modules corresponding to the light paths in the detection region 200 (as shown in FIG. 9A) of the detection apparatus 20 may be increased, so that in the detection region 200 that covers the skin of the user, results of detecting the pulse data of the user by different PPG modules may be obtained. In this case, when some regions in the detection region 200 are affected by the interference source shown in FIG. 8B or noise generated by an electronic component, the processor of the electronic device 01 may compare and analyze, based on detection results of the detection apparatus 20, PPG data (including blood oxygen and heart rates) obtained from different light paths in the detection region 200, to improve detection data accuracy and the signal-to-noise ratio.

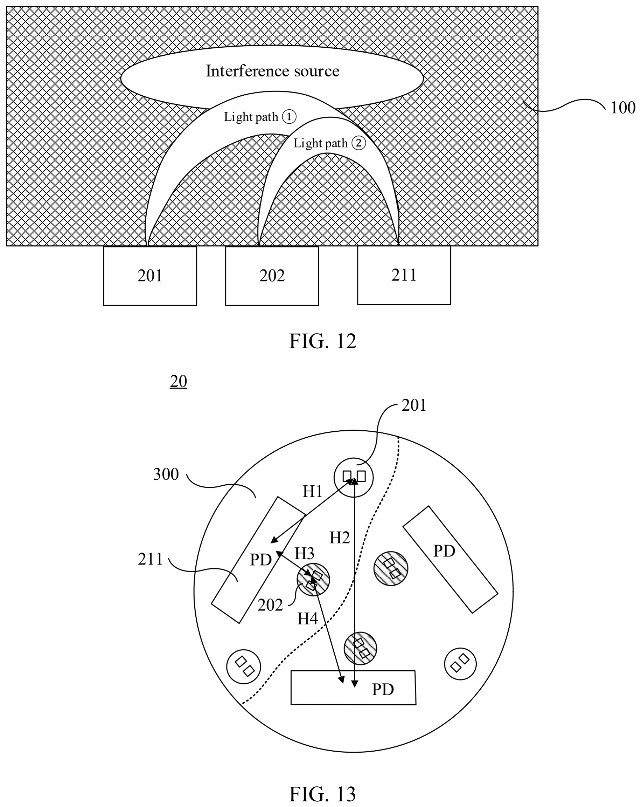

In addition, an effective depth that is in the skin and at which the light emitted by the LED arrives (at the effective depth, a proportion of PPG signals in each layer of the skin is relatively high) varies with a distance between the LED and the PD. For example, as shown in FIG. 11B, a distance between the second light source 202 and the first photodetector 211 is relatively short. Therefore, a depth that is in the skin 100 and at which light (from the first light source 201) in the light path ① arrives may be greater than a depth that is in the skin 100 and at which light (from the second light source 202) in the light path ② arrives. Alternatively, for another example, as shown in FIG. 11C, a distance between the first light source 201 and the first photodetector 211 and a distance between the second light source 202 and the first photodetector 211 are the same or approximately the same. Therefore, a depth that is in the skin 100 and at which light (from the first light source 201) in the light path ① arrives may be the same as or approximately the same as a depth that is in the skin 100 and at which light (from the second light source 202) in the light path ② arrives. In this case, it can be learned from the foregoing that a part that is of the skin 100 and that is used to generate a DC signal serves as an interference source shown in FIG. 12, and causes interference to a signal detected by the PPG module. In addition, the large artery 1041 that can generate the AC signal shown in FIG. 7 is located in the subcutaneous tissue 104 shown in FIG. 6, and a depth of the subcutaneous tissue 104 is greater than depths of the epidermis layer 102 and the dermis layer 103. Therefore, a greater depth that is in the skin and at which light of a light source arrives leads to a higher proportion of an AC signal in obtained detection signals, a higher PI of a detection result, and higher detection accuracy. Therefore, in FIG. 12, because a depth that is in the skin 100 and at which light (from the first light source 201) in the light path ① arrives is greater than a depth that is in the skin 100 and at which light (from the second light source 202) in the light path ② arrives, a PI of a detection signal obtained by the first photodetector 211 in a PPG module corresponding to the light path ① is greater than a PI of a detection signal obtained by the first photodetector 211 in a PPG module corresponding to the light path ②.

It can be learned from the foregoing that, in a light path, a PI of an obtained detection result varies with a depth that is in the skin and at which light arrives. Therefore, to enable the detection apparatus 20 to obtain detection results with different PIs to improve detection result accuracy and the signal-to-noise ratio of the detection apparatus 20, as shown in FIG. 13, in a same distribution region 300, there is a first distance H1 between a first light source 201 and at least one first photodetector 211. For example, when there are two or more first photodetectors 211 in the distribution region 300, in the same distribution region 300, there may be the first distance H1 between the first light source 201 and one of the first photodetectors 211, and a distance between the first light source 201 and a remaining first photodetector 211 may be the same as or different from the first distance H1.

In addition, still as shown in FIG. 13, there is a second distance H2 between the first light source 201 and at least one first photodetector 211 outside the distribution region 300. For example, when there are two or more first photodetectors 211 outside the distribution region 300, there is the second distance H2 between the first light source 201 and one first photodetector 211 outside the distribution region 300, and a distance between the first light source 201 and a remaining first photodetector 211 outside the distribution region 300 may be the same as or different from the second distance H2.

For example, as shown in FIG. 13, in the same distribution region 300, there is the first distance H1 between the first light source 201 and the first photodetector 211. There is the second distance H2 between the first light source 201 and the at least one first photodetector 211 outside the distribution region 300, where H1 < H2. For example, 0 mm < H1 < 4 mm. In this case, in the same distribution region 300, a light path formed between the first light source 201 and the first photodetector 211 may be a light path with a short distance range (less than 4 mm). Alternatively, 4 mm ≤ H1 < 7 mm. In this case, in the same distribution region 300, a light path formed between the first light source 201 and the first photodetector 211 may be a light path with a medium distance range (4 mm to 7 mm). In addition, H2 > 10 mm. In this case, a light path formed between the first light source 201 and the at least one first photodetector 211 outside the distribution region 300 is a light path with an ultra-long distance range (greater than 10 mm).

In addition, still as shown in FIG. 13, in a same distribution region 300, there is a third distance H3 between a second light source 202 and at least one first photodetector 211. For example, when there are two or more first photodetectors 211 in the distribution region 300, in the same distribution region 300, there may be the third distance H3 between the second light source 202 and one of the first photodetectors 211, and a distance between the second light source 202 and a remaining first photodetector 211 may be the same as or different from the third distance H3.

In addition, there is a fourth distance H4 between the second light source 202 and at least one first photodetector 211 outside the distribution region 300. For example, when there are two or more first photodetectors 211 outside the distribution region 300, there is the fourth distance H4 between the second light source 202 and one first photodetector 211 outside the distribution region 300, and a distance between the second light source 202 and a remaining first photodetector 211 outside the distribution region 300 may be the same as or different from the fourth distance H4. Any two of the first distance H1, the second distance H2, the third distance H3, and the fourth distance H4 are different.

For example, as shown in FIG. 13, in the same distribution region 300, there is the third distance H3 between the second light source 202 and the first photodetector 211. There is the fourth distance H4 between the second light source 202 and the at least one first photodetector 211 outside the distribution region 300, where H3 < H4 < H2. For example, when 0 mm < H1 < 4 mm, 4 mm ≤ H3 < 7 mm. In this case, in the same distribution region 300, when the light path formed between the first light source 201 and the first photodetector 211 is a light path with a short distance range (less than 4 mm), in the same distribution region 300, a light path formed between the second light source 202 and the first photodetector 211 may be a light path with a medium distance range (4 mm to 7 mm). Alternatively, when 4 mm ≤ H1 < 7 mm, 0 mm < H3 < 4 mm. In this case, in the same distribution region 300, when the light path formed between the first light source 201 and the first photodetector 211 is a light path with a medium distance range (4 mm to 7 mm), in the same distribution region 300, a light path formed between the second light source 202 and the first photodetector 211 may be a light path with a short distance range (less than 4 mm). In addition, 7 mm ≤ H4 ≤ 10 mm. In this case, a light path formed between the second light source 202 and the at least one first photodetector 211 outside the distribution region 300 may be a light path with a long distance range (7 mm to 10 mm).

On this basis, to make distribution of the first light sources 201, the second light sources 202, and the first photodetectors 211 in the detection apparatus 20 symmetric to improve appearance quality of the electronic device 01, the first distances H1 in different distribution regions may be the same, the second distances H2 in different distribution regions may be the same, the third distances H3 in different distribution regions may be the same, and the fourth distances H4 in different distribution regions may be the same.

Based on this, the detection apparatus 20 may have light paths corresponding to at least four distances (the first distance H1, the second distance H2, the third distance H3, and the fourth distance H4). PIs of PPG data that may be obtained by PPG modules in light paths corresponding to different distances are different. In this way, the processor of the electronic device 01 may compare and analyze PPG data with different PIs based on detection results of the detection apparatus 20, to improve detection data accuracy and the signal-to-noise ratio.

As shown in FIG. 13, a same first photodetector 211 may receive light from different first light sources 201 or different second light sources 202. To enable the processor of the electronic device 01 to distinguish PPG data from different light paths, in the detection apparatus 20, time-sharing driving may be performed between a first light source 201 and a second light source 202, between different first light sources 201, and between different second light sources 202, so that different light sources may be turned on in a time-sharing manner. A manner of time-sharing driving of different light sources is not limited in this application.

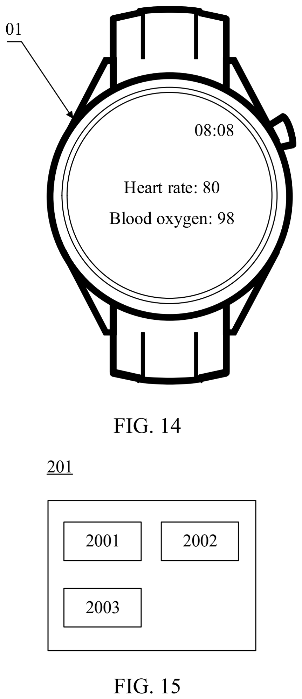

It can be learned from the foregoing that, based on detection results of the detection apparatus 20, the processor of the electronic device 01 may not only compare and analyze PPG data obtained from different light paths in the detection region 200, but also compare and analyze PPG data with different PIs, to obtain final PPG data as a detection result for output. For example, when the electronic device 01 with the detection apparatus 20 has a display function, as shown in FIG. 14, the electronic device 01 may display the PPG data, for example, blood oxygen and a heart rate, so that the user obtains a health detection result in a timely manner.

To obtain the blood oxygen data, in some embodiments of this application, either of the first light source 201 and the second light source 202 may include a first light emitting device 2001 and a second light emitting device 2002 that are shown in FIG. 15. The first light emitting device 2001 may emit red (red, RD) light whose wavelength ranges from 660 nm to 735 nm. The second light emitting device 2002 may emit infrared (infrared, IR) light whose wavelength ranges from 805 nm to 940 nm. The red light is mainly absorbed by deoxygenated hemoglobin in blood, and the infrared light is mainly absorbed by oxygenated hemoglobin in the blood. Therefore, the processor of the electronic device 01 may calculate blood oxygen saturation by measuring a ratio of the deoxygenated hemoglobin to the oxygenated hemoglobin in the blood by using an absorption difference that is between the red light and the infrared light and that is detected by the detection apparatus 20, to obtain the blood oxygen data.

In addition, it can be learned from the foregoing that the heart rate of the user may be determined based on the trough position a2 (corresponding to the systolic phase peak value) and the peak position b2 (corresponding to the diastolic phase peak value) in the AC signal shown in FIG. 7. The AC signal is related to absorption and scattering, by the human body, of light emitted by a light source of the detection apparatus 20. Because human blood has a higher absorption rate for green light, to make heart rate data detected by the detection apparatus 20 more accurate, as shown in FIG. 15, heart rate data may be obtained after light emitted by the first light emitting device 2001 or the second light emitting device 2002 of the detection apparatus 20 is received by the PD. However, either of the first light source 201 and the second light source 202 may include a third light emitting device 2003, and the third light emitting device 2003 emits green light.

In FIG. 15, an example in which the first light source 201 includes the first light emitting device 2001, the second light emitting device 2002, and the third light emitting device 2003 is used for description. The second light source 202 may also include the first light emitting device 2001, the second light emitting device 2002, and the third light emitting device 2003. In addition, FIG. 15 is merely an example for describing arrangement positions of the first light emitting device 2001, the second light emitting device 2002, and the third light emitting device 2003. The arrangement positions of the foregoing light emitting devices are not limited in this application.

In addition, the foregoing description is provided by using an example in which the detection apparatus 20 detects the blood oxygen and the heart rate of the user. In some other embodiments of this application, the processor of the electronic device 01 may further obtain data of the user such as blood pressure, sleep, hemoglobin concentration, a pulse, blood oxygen saturation, a respiratory rate, a blood perfusion index, blood flow reactivity, methemoglobin, carboxyhemoglobin, biliflavin, and oxygen content based on the blood oxygen and heart rate data detected by the detection apparatus 20.

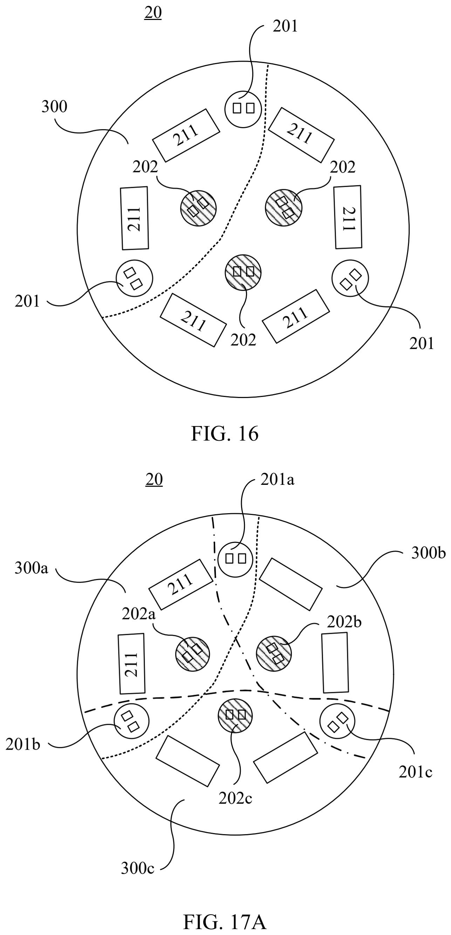

The following describes an example of a structure of the detection apparatus 20 with reference to quantities and arrangement positions of first light sources 201, second light sources 202, and first photodetectors 211 in a distribution region, and quantities of light sources and photodetectors in the entire detection apparatus 20. In some other embodiments of this application, as shown in FIG. 16, the detection apparatus 20 may include three first light sources 201 and six first photodetectors 211, and two first photodetectors 211 are disposed between two adjacent first light sources 201. In addition, two first light sources 201, and two first photodetectors 211 and one second light source 202 that are located between the two first light sources 201 are distributed in a distribution region 300. Similarly, in this way, in the same distribution region, each first light source 201 may form two light paths respectively with the two first photodetectors 211, and the second light source 202 between the two first light sources 201 may form two light paths respectively with the first photodetectors 211, so that a quantity of light paths between two adjacent first light sources 201 may be increased.

On this basis, as shown in FIG. 17A, the detection apparatus 20 may have three distribution regions: a distribution region 300a, a distribution region 300b, and a distribution region 300c. One second light source is distributed in each distribution region. For example, a second light source 202a is distributed in the distribution region 300a, a second light source 202b is distributed in the distribution region 300b, and a second light source 202c is distributed in the distribution region 300c. In addition, in the detection apparatus 20, two adjacent distribution regions share a same first light source. For example, the distribution region 300a and the distribution region 300b share a first light source 201a. The distribution region 300b and the distribution region 300c share a first light source 201c, and the distribution region 300c and the distribution region 300a share a first light source 201b.

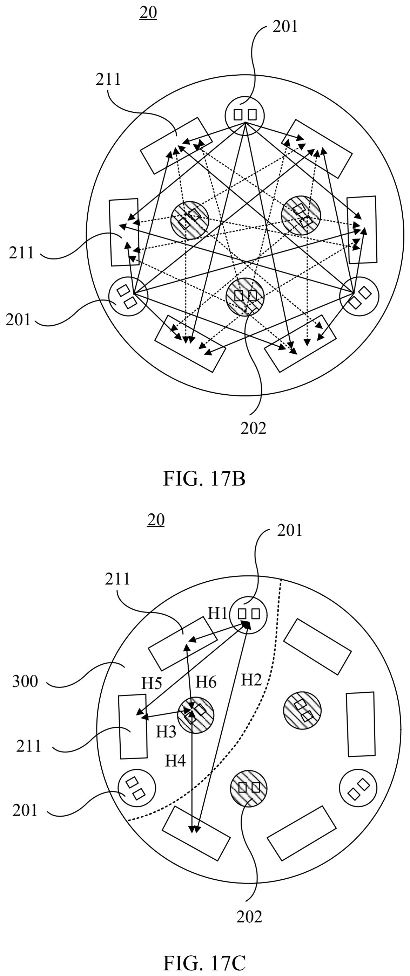

In this case, as shown in FIG. 17B, in the detection apparatus 20, one light path (represented by a solid arrow in FIG. 17B) may be formed between one first light source 201 and one first photodetector 211, and the first light source 201 and the first photodetector 211 that form the light path may form one PPG module. In addition, one light path (represented by a dashed arrow in FIG. 17B) may also be formed between one second light source 202 and one first photodetector 211, and the second light source 202 and the first photodetector 211 that form the light path may form one PPG module.

In this way, when the plurality of first light sources 201 and the plurality of second light sources 202 are driven in a time-sharing manner, as shown in FIG. 17B, each light source (a first light source 201 or a second light source 202) may form one light path (represented by an arrow) with one first photodetector 211. Because distribution positions of the plurality of light sources and the plurality of first photodetectors 211 are different, a plurality of formed light paths intersect with each other in the detection region 200 (as shown in FIG. 9A), thereby increasing density of light paths. Therefore, in the entire detection region 200, an area covered by light paths is larger, and the detection apparatus 20 obtains a larger quantity of PPGs from different light paths, so that impact of interference caused by a relatively large proportion of DC signals in a partial region on a detection result may be reduced, thereby helping improve the detection accuracy.

It can be learned from the foregoing that, in a light path, a PI of an obtained detection result varies with a depth that is in the skin and at which light arrives. Therefore, to enable the detection apparatus 20 to obtain detection results with different PIs to improve detection result accuracy and the signal-to-noise ratio of the detection apparatus 20, for example, as shown in FIG. 17C, in a same distribution region 300, a first light source 201 has a first distance H1 and a fifth distance H5 respectively from two first photodetectors 211, and the first distance H1 and the fifth distance H5 may be different. There is a second distance H2 between the first light source 201 and at least one first photodetector 211 outside the distribution region 300, where H1 < H5 < H2. In addition, in the same distribution region 300, a second light source 202 has a third distance H3 and a sixth distance H6 respectively from the two first photodetectors 211. There is a fourth distance H4 between the second light source 202 and at least one first photodetector 211 outside the distribution region 300, where H3 < H4, and H6 < H4.

In some embodiments of this application, 0 mm < H1 < 4 mm. As shown in FIG. 17C, in the same distribution region 300, a light path formed between the first light source 201 and one of the first photodetectors 211 is a light path with the short distance range. For example, when H1 = 3.2 mm, as shown in Table 1, skin depths that can be reached by the light path with the short distance range include the papillary dermis layer (a signal proportion is 0.21%), the upper vascular plexus (a signal proportion is 1.29%), the reticular dermis layer (a signal proportion is 41.04%), and the lower vascular plexus (a signal proportion is 24.29%) in the dermis layer, and the adipose layer (a signal proportion is 33.16%) in the subcutaneous tissue.

It can be learned from the foregoing that, after light emitted by a light source in a PPG module is incident on tissue layers (including the horny layer, the epidermis layer, the papillary dermis layer, the upper vascular plexus, the reticular dermis layer, the lower vascular plexus, and the adipose layer) at different depths in the skin, the light is absorbed and scattered by some tissue layers. Then, a PD in the PPG module may receive light scattered by some tissue layers in the skin, to generate the detection signal. A signal proportion of a tissue layer (for example, the reticular dermis layer) in the skin is a proportion of a detection signal formed by scattered light received by the PD from the tissue layer (for example, the reticular dermis layer) when the light emitted by the light source in the PPG module penetrates the tissue layer (for example, the reticular dermis layer) in total detection signals received by the PD from various tissue layers in the skin. Therefore, a depth of the tissue layer (for example, the reticular dermis layer) corresponding to the signal proportion is a depth at which the light penetrates the skin.

Therefore, a higher signal proportion may indicate a depth, that is, an effective depth, at which most of the light emitted by the light source in the PPG module penetrates the skin. Based on this, it can be learned from data in Table 1 that, when H1 = 3.2 mm, a proportion (41.04%) of signals obtained from the reticular dermis layer by a PPG module corresponding to the first distance H1 is the highest. Therefore, an effective depth that is in the human skin and that is reached by the light path with the short distance range being the first distance H1 may be the reticular dermis layer.

In addition, 4 mm ≤ H3 < 7 mm, 4 mm ≤ H6 < 7 mm, and 4 mm ≤ H5 < 7 mm. The third distance H3, the fifth distance H5, and the sixth distance H6 may be the same or different, provided that it is ensured that the third distance H3, the fifth distance H5, and the sixth distance H6 are within a range of 4 mm to 7 mm. In this case, as shown in FIG. 17C, in the same distribution region 300, a light path formed between the second light source 202 and any first photodetector 211 is a light path with the medium distance range. In addition, in the same distribution region 300, a light path formed between the first light source 201 and the other first photodetector 211 is a light path with the medium distance range.

For example, when H3, H5, or H6 is 5.5 mm, as shown in Table 1, skin depths that can be reached by the light path with the medium distance range include the papillary dermis layer (a signal proportion is 0.58%), the upper vascular plexus (a signal proportion is 1.16%), the reticular dermis layer (a signal proportion is 23.58%), and the lower vascular plexus (a signal proportion is 16.22%) in the dermis layer, and the adipose layer (a signal proportion is 58.46%) in the subcutaneous tissue. Therefore, a proportion (58.46%) of signals obtained from the reticular dermis layer by a PPG module corresponding to the third distance H3, the fifth distance H5, or the sixth distance H6 is the highest. Therefore, an effective depth that is in the human skin and that is reached by the light path with the medium distance range being the third distance H3, the fifth distance H5, or the sixth distance H6 may be the reticular dermis layer.

Similarly, 7 mm ≤ H4 ≤ 10 mm. As shown in FIG. 17C, a light path formed between the second light source 202 and the at least one first photodetector 211 outside the distribution region 300 is a light path with the long distance range. For example, when H4 = 7 mm, as shown in Table 1, skin depths that can be reached by the light path with the long distance range include the upper vascular plexus (a signal proportion is 1.00%), the reticular dermis layer (a signal proportion is 2.02%), and the lower vascular plexus (a signal proportion is 8.54%) in the dermis layer, and the adipose layer (a signal proportion is 88.44%) in the subcutaneous tissue. Therefore, a proportion (88.44%) of signals obtained from the adipose layer by a PPG module corresponding to the fourth distance H4 is the highest. Therefore, an effective depth that is in the human skin and that is reached by the light path with the long distance range being the fourth distance H4 may be the adipose layer. Similarly, H2 > 10 mm. As shown in FIG. 17C, a light path formed between the first light source 201 and the at least one first photodetector 211 outside the distribution region 300 is a light path with the ultra-long distance range. For example, when H2 = 10.2 mm, as shown in Table 1, skin depths that can be reached by the light path with the ultra-long distance range include the upper vascular plexus (a signal proportion is 0.46%), the reticular dermis layer (a signal proportion is 3.62%), and the lower vascular plexus (a signal proportion is 1.26%) in the dermis layer, and the adipose layer (a signal proportion is 94.66%) in the subcutaneous tissue. Therefore, a proportion (94.66%) of signals obtained from the adipose layer by a PPG module corresponding to the second distance H2 is the highest. Therefore, an effective depth that is in the human skin and that is reached by the light path with the ultra-long distance range being the second distance H2 may be the adipose layer.

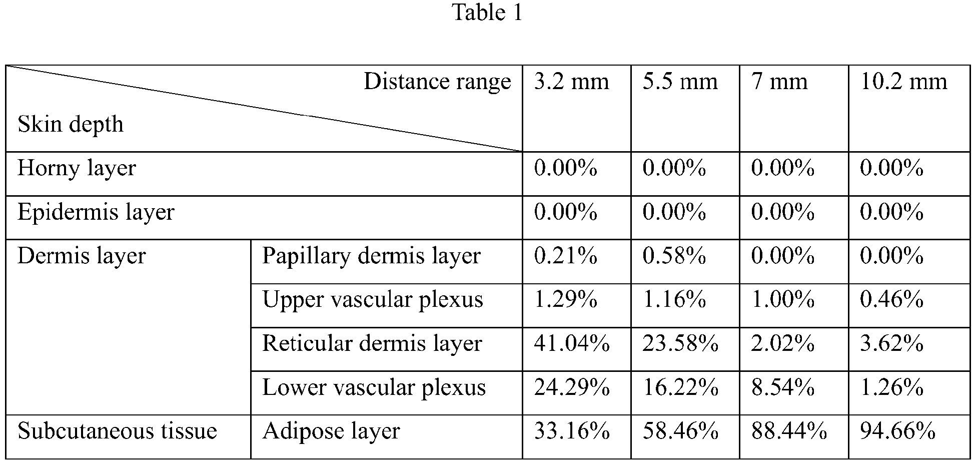

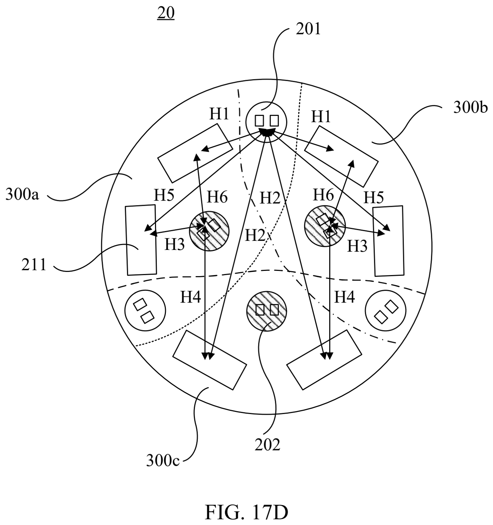

On this basis, to make distribution of the first light sources 201, the second light sources 202, and the first photodetectors 211 in the detection apparatus 20 symmetric to improve appearance quality of the electronic device 01, as shown in FIG. 17D, the first distances H1 in different distribution regions (for example, the distribution region 300a and the distribution region 300b) may be the same, the second distances H2 in different distribution regions (for example, the distribution region 300a and the distribution region 300b) may be the same, the third distances H3 in different distribution regions (for example, the distribution region 300a and the distribution region 300b) may be the same, the fourth distances H4 in different distribution regions (for example, the distribution region 300a and the distribution region 300b) may be the same, the fifth distances H5 in different distribution regions may be the same, and the sixth distances H6 in different distribution regions may be the same.

The foregoing describes an example of a manner of setting the first distance H1, the second distance H2, the third distance H3, the fourth distance H4, the fifth distance H5, and the sixth distance H6 in the distribution region 300a and the distribution region 300b. In some other embodiments of this application, a manner of setting the foregoing distances in the distribution region 300a and the distribution region 300c, or in the distribution region 300b and the distribution region 300c is the same as that described above. Details are not described herein again.

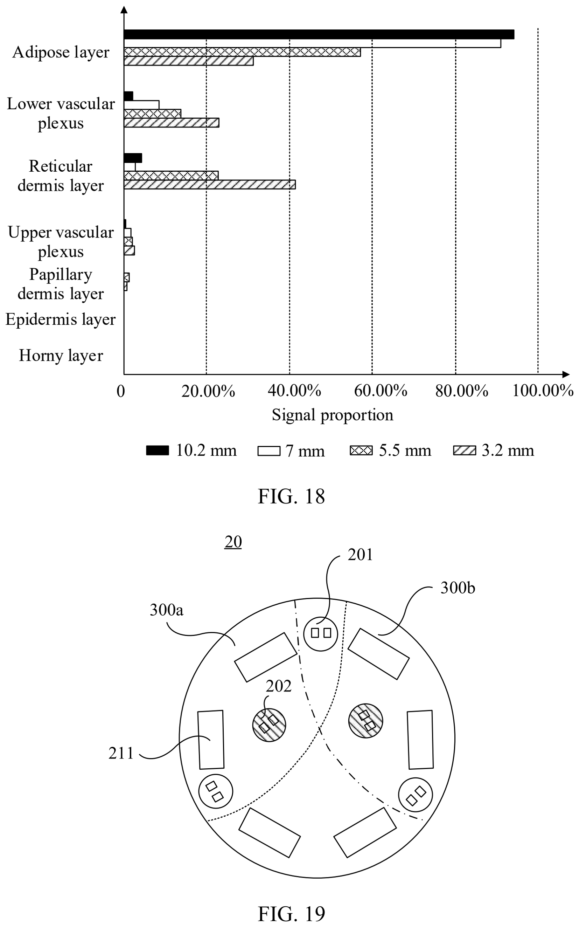

In addition, a bar chart shown in FIG. 18 may be obtained by sorting the data in Table 1. It can be learned from FIG. 18 that, when the detection apparatus 20 drives the first light sources 201 and the second light sources 202 in a time-sharing manner, almost none of detection signals obtained from light paths with various distance ranges are from the horny layer and the epidermis layer, and a relatively small proportion, approximately 1.00%, of the detection signals are from the papillary dermis layer and the upper vascular plexus. In addition, a relatively high proportion of detection signals obtained from the light path with the long distance range (7 mm to 10 mm) and the light path with the medium distance range (4 mm to 7 mm) are from the lower vascular plexus and the reticular dermis layer. A relatively high proportion of detection signals obtained from the light path with the long distance range (7 mm to 10 mm) and the light path with the ultra-long distance range (greater than 10 mm) are from the adipose layer.

A highest proportion of detection signals obtained from the light path with the ultra-long distance range (greater than 10 mm) are from the adipose layer. It can be learned from the foregoing that the large artery 1041 (as shown in FIG. 6) is distributed in the subcutaneous tissue 104 in which the adipose layer is located, and the pulsatile component of artery blood of the large artery 1041 can generate the AC signal, so that a signal obtained by the detection apparatus 20 has a higher perfusion index. For example, when the detection apparatus 20 detects blood oxygen, light emitting devices in a light source of the detection apparatus 20 include the first light emitting device 2001 that emits RD light and the second light emitting device 2002 that emits IR light (as shown in FIG. 15). When the light path with the ultra-long distance range (greater than 10 mm) is used, a red light perfusion index PI_RD obtained by the detection apparatus 20 may reach 0.24%, and an infrared light perfusion index PI_RD obtained by the detection apparatus 20 may reach 0.36%. In addition, it can be further learned from Table 2 that, when the light path with the long distance range (7 mm to 10 mm), the light path with the medium distance range (4 mm to 7 mm), and the light path with the short distance range (less than 4 mm) are separately used, the red light perfusion index PI_RD and the infrared light perfusion index PI_RD obtained by the detection apparatus 20 sequentially decrease.

Based on this, when health detection is performed on different users, due to different physical conditions of human bodies, under a same detection condition (for example, a detection temperature or a distance of a light path), perfusion indexes of detection data of some users are relatively low, and the users may be referred to as low-perfusion index individuals (for example, elderly individuals, females, or individuals with relatively thin bodies). In addition, a blood flow status of a human body is related to an ambient temperature. When the ambient temperature decreases, a perfusion index of obtained detection data also decreases. To resolve the foregoing problem, when the detection apparatus 20 provided in this embodiment of this application is used, the first light sources 201 and the second light sources 202 may be driven in a time-sharing manner, to detect the user by using the light path with the ultra-long distance range (greater than 10 mm), so that more light emitted by the light sources can reach the adipose layer, thereby obtaining detection data with a relatively high perfusion index.

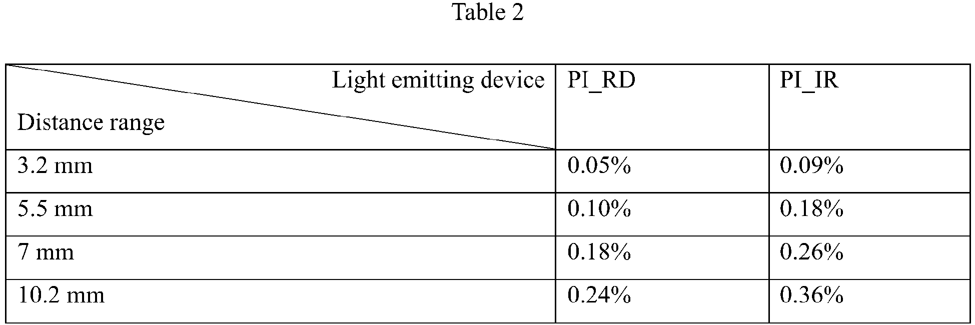

Alternatively, in some other embodiments of this application, 0 mm < H3 < 4 mm. In this case, as shown in FIG. 17C, in the same distribution region 300, a light path formed between the second light source 202 and one of the first photodetectors 211 is a light path with the short distance range. Similarly, an effective depth that is in the human skin and that is reached by the light path with the short distance range may be the reticular dermis layer. In addition, 4 mm ≤ H1 ≤ 7 mm, and 4 mm ≤ H6 < 7 mm. Similarly, the first distance H1 and the sixth distance H6 may be the same or different. In this case, in the same distribution region 300, a light path formed between the first light source 201 and one of the first photodetectors 211 is a light path with the medium distance range. In the same distribution region 300, a light path formed between the second light source 202 and the other first photodetector 211 is a light path with the medium distance range. Similarly, an effective depth that is in the human skin and that is reached by the light path with the medium distance range may be the reticular dermis layer.

In addition, 7 mm < H4 ≤ 10 mm, and 7 mm < H5 ≤ 10 mm. As shown in FIG. 17C, a light path formed between the second light source 202 and the at least one first photodetector 211 outside the distribution region 300 is a light path with the long distance range. In addition, in the same distribution region 300, a light path formed between the first light source 201 and the other first photodetector 211 is a light path with the long distance range. H2 > 10 mm, and a light path formed between the first light source 201 and the at least one first photodetector 211 outside the distribution region 300 is a light path with the ultra-long distance range. Similarly, an effective depth that is in the human skin and that is reached by the light path with the long distance range and the light path with the ultra-long distance range may be the adipose layer. Technical effects of the distances are the same as those described above. Details are not described herein again.

It can be learned from the foregoing that the detection apparatus 20 may have light paths with four distance ranges: the light path with the short distance range (less than 4 mm), the light path with the medium distance range (4 mm to 7 mm), the light path with the long distance range (7 mm to 10 mm), and the light path with the ultra-long distance range (greater than 10 mm). PIs of PPG data that may be obtained by PPG modules in light paths corresponding to different distance ranges are different. In this way, the processor of the electronic device 01 may compare and analyze PPG data with different PIs based on detection results of the detection apparatus 20, to improve detection data accuracy and the signal-to-noise ratio. For example, as shown in Table 3, that the detection apparatus 20 detects blood oxygen data is used as an example. When the light paths with the foregoing four distance ranges are used, in collected blood oxygen data, a proportion of abnormal data generated due to interference from the foregoing interference source is 0.17%, blood oxygen detection accuracy (rms) is 4.23, and data detection accuracy is relatively high. A smaller value of the blood oxygen detection accuracy (rms) indicates higher accuracy.

In comparison, when the detection apparatus has only light paths with three distance ranges, for example, the light path with the short distance range (less than 4 mm), the light path with the medium distance range (4 mm to 7 mm), and the light path with the long distance range (7 mm to 10 mm), as shown in Table 3, in collected blood oxygen data, a proportion of abnormal data generated due to interference from the foregoing interference source increases to 1.63%, the blood oxygen detection accuracy (rms) increases to 5.13, and the data detection accuracy decreases. Alternatively, when the detection apparatus has only light paths with three distance ranges, for example, the light path with the short distance range (less than 4 mm), as shown in Table 3, in collected blood oxygen data, a proportion of abnormal data generated due to interference from the foregoing interference source increases to 11.50%, the blood oxygen detection accuracy (rms) increases to 6.53, and the data detection accuracy decreases. Therefore, a larger quantity of different distance ranges that the light paths of the detection apparatus 20 have leads to higher accuracy of obtained health detection data.

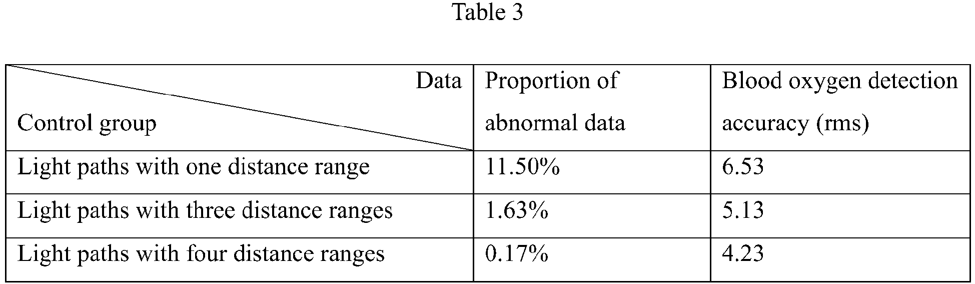

The foregoing description is provided by using an example in which the detection apparatus 20 includes three second light sources 202. In some other embodiments of this application, as shown in FIG. 19, the detection apparatus 20 may include two second light sources 202. Based on this, the detection apparatus 20 may have two distribution regions: a distribution region 300a and a distribution region 300b. There is one second light source 202 in each distribution region. The distribution region 300a and the distribution region 300b that are adjacent to each other share a same first light source 201.

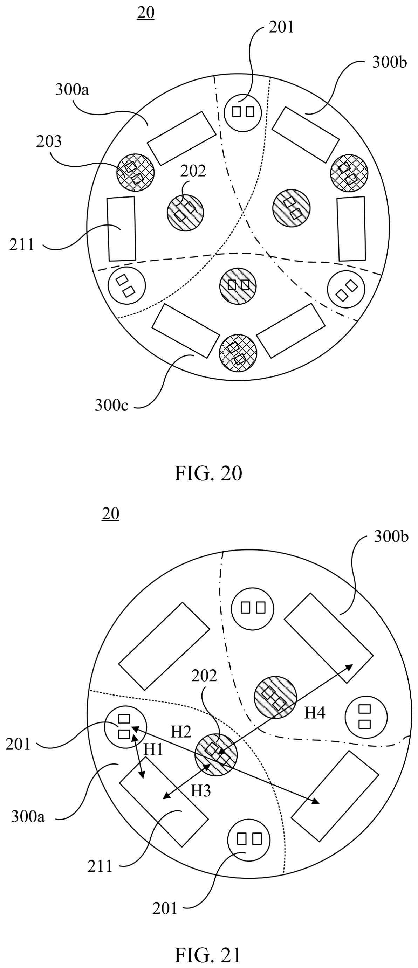

In some other embodiments of this application, as shown in FIG. 20, when the detection apparatus 20 has three first light sources 201, six first photodetectors 211, and three second light sources 202, the detection apparatus 20 may further include three third light sources 203 (represented by cross-filled patterns in the figure). One third light source 203 is disposed between two connected first photodetectors 211. In this way, each third light source 203 may form two light paths respectively with two first photodetectors 211 adjacent to the third light source 203, so that a quantity of light paths between the two adjacent first photodetectors 211 may be increased, to improve the detection accuracy. In FIG. 20, an example in which the detection apparatus 20 includes three second light sources 202 is used for description. In some other embodiments of this application, the detection apparatus 20 may alternatively include two second light sources 202. Correspondingly, the detection apparatus 20 has two distribution regions, and one second light source 202 is disposed in each distribution region.

In some other embodiments of this application, as shown in FIG. 21, the detection apparatus 20 may include four first light sources 201 and four first photodetectors 211, and one first photodetector 211 is disposed between two adjacent first light sources 201. In addition, two first light sources 201, one first photodetector 211, and one second light source 202 are distributed in a distribution region, for example, a distribution region 300a. Technical effects of the first light source 201, the second light source 202, and the first photodetector 211 are the same as those described above. Details are not described herein again.

For example, still as shown in FIG. 21, in the same distribution region 300a, there is a first distance H1 between the first light source 201 and the first photodetector 211, and there is a second distance H2 between the first light source 201 and at least one first photodetector 211 outside the distribution region 300a. In the same distribution region 300a, there is a third distance H3 between the second light source 202 and the first photodetector 211. There is a fourth distance H4 between the second light source 202 and at least one first photodetector 211 outside the distribution region 300a. Setting manners and technical effects of the first distance H1, the second distance H2, the third distance H3, and the fourth distance H4 are the same as those described above. Details are not described herein again.

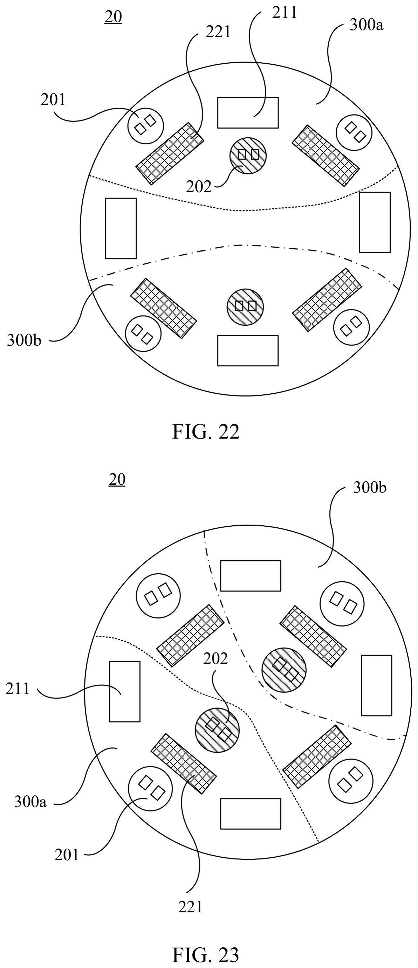

In some other embodiments of this application, as shown in FIG. 22, the detection apparatus 20 may include four first light sources 201 and four first photodetectors 211, and one first photodetector 211 is disposed between two adjacent first light sources 201. In addition, the detection apparatus 20 may further include four second photodetectors 221, and one second photodetector 221 is disposed between two adjacent first photodetectors 211. In this case, one second photodetector 221 and one first light source 201 are disposed between two adjacent first photodetectors 211.

Based on this, the detection apparatus 20 has two distribution regions (a distribution region 300a and a distribution region 300b), the detection apparatus 20 may include two second light sources 202, and one second light source 202 is located in one of the foregoing distribution regions (the distribution region 300a and the distribution region 300b). In addition, two second photodetectors 221 and one first photodetector 211 are distributed in a distribution region, for example, the distribution region 300a. In this way, in the same distribution region, either of the first light source 201 and the second light source 202 may not only form a light path with the first photodetector 211, but also form a light path with the second photodetector 221, thereby increasing a quantity of light paths. In addition, a manner of setting a distance between the light source and each photodetector may be similarly obtained. Details are not described herein again.

In some other embodiments of this application, as shown in FIG. 23, the detection apparatus 20 may further include four second photodetectors 221, and the second photodetectors 221 are disposed in the detection region 200 (as shown in FIG. 9A). In addition, the detection apparatus 20 may include four first light sources 201, four first photodetectors 211, and two second light sources 202, and one first photodetector 211 is disposed between two adjacent first light sources 201. The detection apparatus has two distribution regions, for example, a distribution region 300a and a distribution region 300b.

Two first photodetectors 211, and one second photodetector 221, one second light source 202, and one first light source 201 that are located between the two first photodetectors 211 are distributed in any distribution region, for example, the distribution region 300a, and the second photodetector 221 is located between the first light source 201 and the second light source 202. The detection apparatus has two distribution regions 300, and one second light source 202 is located in one distribution region 300. In this way, in the same distribution region, either of the first light source 201 and the second light source 202 may not only form a light path with the first photodetector 211, but also form a light path with the second photodetector 221, thereby increasing a quantity of light paths. In addition, a manner of setting a distance between the light source and each photodetector may be similarly obtained. Details are not described herein again.

The foregoing descriptions are merely specific implementations of this application, but are not intended to limit the protection scope of this application. Any variation or replacement within the technical scope disclosed in this application shall fall within the protection scope of this application. Therefore, the protection scope of this application shall be subject to the protection scope of the claims.

## Claims

1. A detection apparatus, comprising:
at least three first light sources;
at least three first photodetectors, wherein at least one first photodetector is disposed between two adjacent first light sources, and a detection region is enclosed by the at least three first light sources and the at least three first photodetectors; and
at least two second light sources, disposed in the detection region, wherein
the detection apparatus has at least two distribution regions, and the first light sources, the second light sources, and the first photodetectors are distributed in the distribution regions; and
in a same distribution region, there is a first distance H1 between the first light source and at least one first photodetector; there is a second distance H2 between the first light source and at least one first photodetector outside the distribution region; in a same distribution region, there is a third distance H3 between the second light source and at least one first photodetector; there is a fourth distance H4 between the second light source and at least one first photodetector outside the distribution region; and any two of the first distance H1, the second distance H2, the third distance H3, and the fourth distance H4 are different.

2. The detection apparatus according to claim 1, wherein
0 mm < H1 < 4 mm, 4 mm ≤ H3 < 7 mm, 7 mm ≤ H4 ≤ 10 mm, and H2 > 10 mm;
or
0 mm < H3 < 4 mm, 4 mm ≤ H1 < 7 mm, 7 mm ≤ H4 ≤ 10 mm, and H2 > 10 mm.

3. The detection apparatus according to claim 1 or 2, wherein
two first light sources, and one second light source and at least one first photodetector that are located between the two first light sources are distributed in the distribution region.

4. The detection apparatus according to claim 3, wherein
the detection apparatus comprises three first light sources and six first photodetectors, two first photodetectors are disposed between two adjacent first light sources, and two first photodetectors are distributed in the distribution region.

5. The detection apparatus according to claim 4, wherein
in a same distribution region, the first light source has the first distance H1 and a fifth distance H5 respectively from two first photodetectors, and the first distance H1 and the fifth distance H5 are different; and
in a same distribution region, the second light source has the third distance H3 and a sixth distance H6 respectively from two first photodetectors.

6. The detection apparatus according to any one of claims 2 to 5, wherein
the first distances H1 in different distribution regions are the same;
the second distances H2 in different distribution regions are the same;
the third distances H3 in different distribution regions are the same; and
the fourth distances H4 in different distribution regions are the same.

7. The detection apparatus according to claim 3, wherein
the detection apparatus comprises three first light sources and three first photodetectors, one first photodetector is disposed between two adjacent first light sources, and one first photodetecting is distributed in the distribution region.

8. The detection apparatus according to any one of claims 4 to 7, wherein
the detection apparatus has three distribution regions, the detection apparatus comprises three second light sources, and one second light source is located in one distribution region; and
two adjacent distribution regions share a same first light source.

9. The detection apparatus according to any one of claims 4 to 7, wherein
the detection apparatus has two distribution regions, the detection apparatus comprises two second light sources, and one second light source is located in one distribution region; and
two adjacent distribution regions share a same first light source.

10. The detection apparatus according to any one of claims 4 to 6, wherein
the detection apparatus further comprises three third light sources, and one third light source is disposed between two adjacent first photodetectors.

11. The detection apparatus according to claim 3, wherein
the detection apparatus comprises four first light sources, four first photodetectors, and two second light sources; one first photodetector is disposed between two adjacent first light sources; one first photodetector is distributed in the distribution region; and the detection apparatus has two distribution regions, and one second light source is located in one distribution region.

12. The detection apparatus according to claim 3, wherein
the detection apparatus comprises four first light sources and four first photodetectors, and one first photodetector is disposed between two adjacent first light sources;
the detection apparatus further comprises four second photodetectors, and one second photodetector is disposed between two adjacent first photodetectors;
two second photodetectors and one first photodetector are distributed in the distribution region; and
the detection apparatus has two distribution regions, the detection apparatus comprises two second light sources, and one second light source is located in one distribution region.

13. The detection apparatus according to claim 1 or 2, wherein
the detection apparatus further comprises four second photodetectors, and the second photodetectors are disposed in the detection region;
the detection apparatus comprises four first light sources, four first photodetectors, and two second light sources, and one first photodetector is disposed between two adjacent first light sources;
two first photodetectors, and one second photodetector, one second light source, and one first light source that are located between the two first photodetectors are distributed in the distribution region, and the second photodetector is located between the first light source and the second light source; and
the detection apparatus has two distribution regions, and one second light source is located in one distribution region.

14. The detection apparatus according to any one of claims 1 to 13, wherein either of the first light source and the second light source comprises a first light emitting device and a second light emitting device, the first light emitting device emits red light, and the second light emitting device emits infrared light.

15. The detection apparatus according to claim 14, wherein either of the first light source and the second light source further comprises a third light emitting device, and the third light emitting device emits green light.

16. The detection apparatus according to any one of claims 1 to 15, wherein the detection apparatus further comprises:
a transparent cover, covering the first light source, the first photodetector, and the second light source;
an optical film layer, disposed on a side that is of the transparent cover and that faces the first light source;
an ink layer, disposed between the optical film layer and the transparent cover, wherein a first transparent hole, a second transparent hole, and a third transparent hole are provided on the ink layer, the first transparent hole exposes a light emitting surface of the first light source, the second transparent hole exposes a light emitting surface of the second light source, and the third transparent hole exposes a light receiving surface of the first photodetector; and
a light shielding member, disposed on a side that is of the optical film layer and that faces away from the transparent cover, wherein the light shielding member is located between any two of the first light source, the second light source, and the first photodetector.

17. An electronic device, comprising:
a circuit board; and
the detection apparatus according to any one of claims 1 to 16, wherein the detection apparatus is disposed on the circuit board.
